# EUROPÄISCHE PATENTSCHRIFT

(11) **EP 0 384 303 B1**
(45) Veröffentlichungstag und Bekanntmachung des Hinweises auf die Patenterteilung: **03.11.1993**
(21) Anmeldenummer: 90102988.4
(22) Anmeldetag: 16.02.1990
(51) Int. Cl.: C12P 21/06, A23J 3/34

(54) **Verfahren zur Herstellung von Protein-Hydrolysaten mit niedrigem Gehalt an Bitterstoffen**
Method for the production of protein hydrolysates with a low bitter ingredient content
Méthode de production d'hydrolysates de protéine à faible contenu de matières amères

(30) Priorität: 21.02.1989 DE 3905194
(43) Veröffentlichungstag der Anmeldung: 29.08.1990
(73) Patentinhaber: Röhm GmbH, 64293 Darmstadt (DE)
(72) Erfinder: Sprössler, Bruno, Dr., D-6101 Rossdorf 1 (DE); Plainer, Hermann, Dr., D-6107 Reinheim 1 (DE)

(56) Entgegenhaltungen:
- EP-A- 0 223 560
- EP-A- 0 320 717
- DE-A- 1 442 144
- DE-A- 3 518 828
- FR-A- 2 022 408

## Beschreibung

Die Erfindung betrifft ein Verfahren zur Herstellung von Protein-Hydrolysaten mit niedrigem Gehalt an Bitterstoffen durch Einwirkung einer Proteinase und einer mikrobiellen Aminopeptidase.

### Stand der Technik

Protein-Hydrolysate aus schwer verdaulichem oder schwer löslichem tierischem oder pflanzlichem Eiweiß, wie Molke- oder Sojaproteinen, könnten in der Nahrungsmittelindustrie breite Anwendung finden, wenn sie nicht durch einen Gehalt an Bitterstoffen einen unangenehmen Geschmack hätten. Es sind schon zahlreiche Versuche unternommen worden, diesen Bittergeschmack zu beseitigen, jedoch hat keines der entwickelten Verfahren technische Anwendung gefunden.

Nach EP 41 723 werden die Bitterstoffe aus dem Protein-Hydrolysat mit überkritschem Kohlendioxyd extrahiert oder nach US 4 130 555 an einen Ionenaustauscher gebunden. Gemäß US 4 293 583 und EP 14 362 soll es möglich sein, die Bitterstoffe an zerkleinertem Pflanzenmaterial, wie Johannisbrotkernschalen, zu absorbieren, jedoch geht dabei ein beträchtlicher Teil des Protein-Hydrolysats verloren.

Nach neueren Erkenntnissen beruht der Bittergeschmack auf einem Gehalt an Oligopeptiden, die bei fortschreitender Spaltung der nativen Proteine durch Endopeptidasen gebildet werden. Der Bittergeschmack tritt erst nach einem bestimmten Hydrolysegrad, bezogen auf die enthaltenen Peptid-Bindungen, auf, der als Bitterpunkt bezeichnet wird. Der Bitterpunkt hängt stark von dem hydrolysierten Protein ab. Es ist vorgeschlagen worden, die Hydrolyse jeweils vor dem Erreichen des Bitterpunktes abzubrechen, wodurch allerdings die angestrebte verbesserte Verwertbarkeit nicht erreicht wird.

In der EP-A 223 560 ist vorgeschlagen worden, die bitterschmeckenden Oligopeptide mittels einer Aminopeptidase abzubauen. Die Einwirkung der Aminopeptidase kann mit der der Endoprotease einhergehen oder nach dieser erfolgen. Für das Verfahren wird eine Aminopeptidase aus einer Kultur von Streptomyces lactis verwendet, die die zusätzliche vorteilhafte Eigenschaft hat, einen angenehmen Fleisch- oder Käsegeschmack zu erzeugen. Diese Peptidase ist im Handel erhältlich, hat jedoch die Nachteile, daß sie für ein technisches Verfahren zu teuer ist, und vor allem daß sie von einem Mikroorganismus abstammt, der in lebensmitteltoxikologischer Hinsicht nicht als unbedenklich gilt.

Aus der DE-A 1 442 144 ist eine L-Leucinamid, Hypertensin und Oxytocin spaltende Aminopeptidase bekannt, die aus Kulturen bestimmter Aspergillus-Stämme durch fraktionierte Fällung und thermische Inaktivierung von begleitenden Endopeptidasen in Gegenwart von Kobaltsalzen isoliert werden kann. Die erhaltene Aminopeptidase wird für pharmakologische oder analytische Zwecke vorgeschlagen, kommt jedoch für die Lebensmittelindustrie wegen der zur Herstellung verwendeten Schwermetallverbindung nicht in Betracht.

### Aufgabe und Lösung

Aufgabe der Erfindung war die Entwicklung eines im technischen Maßstab wirtschaftlich durchführbaren Verfahrens zur Herstellung von Protein-Hydrolysaten mit einem so niedrigen Gehalt an Bitterstoffen, daß die Verwendung für die Herstellung von Lebensmitteln möglich ist. Der Einsatz von toxikologisch unbedenklichen Enzymen ist dafür eine unabdingbare Voraussetzung. Solche Enzyme werden vorwiegend aus Stämmen der Gattung Aspergillus, insbesondere Asp.oryzae gewonnen.

Gegenstand der Erfindung ist ein Verfahren zur Herstellung von Protein-Hydrolysaten durch Einwirkung einer Proteinase und einer mikrobiellen Aminopeptidase auf ein Protein, bei dem eine Aminopeptidase eingesetzt wird, die aus einem Peptidasen-Gemisch aus einer Aspergillus-Kultur durch mindestens 50 %ige Inaktivierung der begleitenden Endopeptidasen in einer wäßrigen Lösung mit einem pH-Wert zwischen 5 und 9 bei einer Temperatur von 50 bis 80°C erhalten worden ist.

Wird eine wäßrige Caseinlösung mit einem herkömmlichen Proteinase-Präparat aus Asp.oryzae hydrolysiert, so wird bereits bei einem Hydrolysegrad von 1,2 % der Bitterpunkt erreicht. Isoliert man jedoch aus dem gleichen Proteinase-Präparat die darin enthaltene Aminopeptidase, indem man die begleitende Proteinase thermisch inaktiviert, und setzt die isolierte Aminopeptidase zusammen mit der ursprünglichen Proteinase zur Protein-Hydrolyse ein, so wird der Bitterpunkt erst bei einem Hydrolysegrad von 4,4 % erreicht, wo schon eine wesentlich verbesserte Verwertbarkeit erreicht wird.

### Wirkungsweise der eingesetzten Peptidasen

Toxikologisch unbedenkliche Mikroorganismen finden sich in der Gattung Aspergillus vor allem in den technisch verwendeten Stämmen der Arten Asp.oryzae, niger, sojae, melleus, und awamori. Protease bildende Stämme dieser Gruppe sind dem Fachmann bekannt. Die meisten Protease-Bildner erzeugen gleichzeitig Exo- und Endopeptidasen. Endopeptidasen spalten Proteinmoleküle an beliebigen Stellen innerhalb der Polymerkette, so daß bei fortschreitender Einwirkung immer kürzere Polypeptid-Bruchstücke entstehen, darunter auch die Bitterpeptide. Dagegen spalten Exopeptidasen einzelne Aminosäuren oder kurze Peptidstücke, die wenig Bittergeschmack besitzen, vom Kettenende der nativen Proteine oder der Polypeptid-Bruchstücke ab. Das natürliche Mischungsverhältnis der Exopeptidasen, die Polypeptidketten vorwiegend vom Aminoende her spalten und daher als Aminopeptidasen bezeichnet werden, zu den Endopeptidasen, die üblicherweise als Proteinasen bezeichnet werden, ist stets weit zugunsten der letzteren verschoben. Bei der Einwirkung des natürlichen Peptidasengemisches auf Proteine eilt daher die Bildung der Polypeptidbruchstücke ihrem Abbau durch Exopeptidasen voraus.

Beim Verfahren der Erfindung wird entweder die Aminopeptidase allein auf das bereits mittels der Proteinase zu Bruchstücken abgebaute Protein-Hydrolysat einwirken gelassen oder beide Enzyme werden gleichzeitig einwirken gelassen, wobei jedoch das Mischungsverhältnis so gewählt wird, daß die Proteinase-Wirkung der Aminopeptidase-Wirkung nicht vorauseilt. Im letztgenannten Fall ist es nicht erforderlich, daß der natürliche Anteil der Proteinase vollständig inaktiviert worden ist. Es genügt häufig, wenn er auf die Hälfte, besser auf 5 bis 25 des ursprünglichen Gehalts inaktiviert worden ist. Technisch ist es einfacher, den Proteinase-Anteil vollständig zu inaktivieren und - sofern eine einstufige Hydrolyse durchgeführt werden soll - die erforderliche Proteinase-Menge in Form des ursprünglichen Enzympräparats oder einer gleichwertigen Proteinase wieder zuzusetzen.

Vorzugsweise wird ein Peptidase-Präparat eingesetzt, welches auf 100.000 Peptidase-Einheiten 0 bis 0,3 Anson-Einheiten aufweist. Die Anson-Einheiten werden nach Anson, Journ.Gen.Physiol., Vol.22, S.79 (1938) bestimmt. Eine Aminopeptidase-Einheit ist definiert als die Enzymmenge, die in einer 0,0006-molaren wäßrigen Lösung von L-Leucin-p-nitranilid bei pH 7,0 und 30°C eine Hydrolysegeschwindigkeit von 1 Mikromol pro Minute bewirkt.

Um eine hohe Ausbeute an Aminopeptidase zu erreichen, ist es vorteilhaft, einen Stamm einer Aspergillus-Art zu wählen, der sich durch eine hohe Aminopeptidase-Produktivität auszeichnet. Solche Stämme lassen sich an ihrer Spaltungsaktivität gegenüber L-Leucin-p-nitranilid und L-Cystin-bis-p-nitranilid erkennen; dazu eignet sich außer der oben angegebenen Bestimmungsmethode ein von H. Tuppy, U. Wiesbauer und E. Wintersberger in Hoppe-Seylers Zeitschrift für physiologische Chemie, Band 329 (1962) auf den Seiten 278 bis 288 beschriebenes Testverfahren.

### Herstellung der Aminopeptidase

Als Ausgangsmaterial wird das aus einer Aspergillus-Kultur gewonnene Kulturfiltrat eingesetzt, vorzugsweise von Asp.oryzae. Das in üblicher Weise erzeugte Kulturfiltrat oder eine durch Fällung und Lösen in Wasser erhaltene Enzymlösung wird zwecks besserer selektiver Stabilisierung vorzugsweise mit wenigstens 10, bevorzugt 40 bis 60 Gew.-%, bezogen auf das Gewicht des Kulturfiltrats oder der wäßrigen Lösung, mit einer Polyhydroxyverbindung versetzt. Bewährt haben sich beispielsweise Glycerin, Glucose, Saccharose und Sorbit. Die Mischung wird auf einen pH-Wert zwischen 5 und 9, vorzugsweise 6 bis 7 eingestellt. Zur Inaktivierung wird sie für 0,1 bis 48 Stunden auf eine Temperatur von 50 bis 80°C erhitzt, wobei die Erhitzungsdauer umso kürzer sein kann, je höher die Temperatur liegt. Optimal ist eine Erhitzung innerhalb 30 bis 60 min auf 60 bis 80°C und Abkühlung nach etwa einer weiteren Stunde. Bei dieser Behandlung nimmt die Aktivität der Proteinase auf 0 bis 10 % des ursprünglichen Gehalts ab, während die Aminopeptidase-Aktivität nahezu vollständig erhalten bleibt.

Die erhaltene flüssige Aminopeptidase-Lösung ist lagerfähig und kann als solche bei der Protein-Hydrolyse eingesetzt werden. Man kann wahlweise auch das Enzym ausfällen, abfiltrieren und in bekannter Weise zu einem Pulverprodukt aufarbeiten.

### Herstellung von Protein-Hydrolysaten

Das Verfahren der Erfindung eignet sich zur Hydrolyse von zahlreichen tierischen oder pflanzlichen Eiweißstoffen zu wertvollen Protein-Hydrolysaten, die geschmacksneutral oder wohlschmeckend und im wesentlichen frei von Bittergeschmack sind. Beispiele hydrolysierbarer Proteine sind Soja-Proteine, Gluten, Getreide- und Bohnenproteine, Kartoffenprotein, Hefeproteine und andere mikrobielle Proteine, Hämoglobin und Fleisch- und Fischproteinmaterial. Vorzugsweise wird das Verfahren zur Hydrolyse von Milchproteinen, insbesondere Casein und Molkenprotein, eingesetzt.

Als Proteinase wird - vor allem bei einstufiger Arbeitsweise - zweckmäßig das gleiche Peptidasen-Gemisch verwendet, aus dem die Aminopeptidase durch das beschriebene Inaktivierungsverfahren hervorgegangen ist. Es kann jedoch auch jede andere wirksame Proteinase verwendet werden, beispielsweise mikrobielle, pflanzliche oder pankreatische Proteinasen. Dabei sind Aminopeptidasereiche Proteinasen bevorzugt.

Die Hydrolyse wird am besten im Temperaturbereich von 10 bis 60, vorzugsweise 25 bis 45°C, in 1 bis 10 Stunden unter Rühren durchgeführt. Zweckmäßig werden die Ausgangsproteine in einer wäßrigen Aufschlemmung oder Lösung mit einem Feststoffgehalt von 5 bis 15 Gew.-% eingesetzt. Der pH-Wert liegt im allgemeinen im Bereich von 5 bis 9,5, vorzugsweise bei 6 bis 8.

Das Hydrolyse-Verfahren kann ein- oder zweistufig durchgeführt werden. Bei der zweistufigen Arbeitsweise läßt man in der ersten Stufe eine herkömmliche Proteinase bis zu einem Hydrolysegrad einwirken, bei dem der Bitterpunkt überschritten ist. Der anzustrebende Hydrolysegrad richtet sich nach der Natur des eingesetzten Proteins und der angestrebten Funktionalität wie Aufschlagfähigkeit, Emulgierfähigkeit, oder Viskosität. Für die Anwendung als Diät- und Babynahrung wird auf einen möglichst hohen Hydrolysegrad Wert gelegt. In der zweiten Stufe läßt man zum Abbau der Bitterstoffe die Aminopeptidase einwirken. Die Verfahrensbedingungen sind in der Regel in beiden Stufen im wesentlichen gleich.

Vorzugsweise wird das Verfahren einstufig durchgeführt, indem man die Proteinase und die Aminopeptidase gleichzeitig einwirken läßt. Auch in diesem Falle wird die Hydrolyse über den Hydrolysegrad hinausgeführt, bei dem bei alleinigem Einsatz der Proteinase der Bitterpunkt erreicht worden wäre. Zweckmäßig werden je 100 Gramm des zu verarbeitenden Proteins 0,1 bis 10 Anson-Einheiten an Proteinase und 10⁵ bis 10⁷ Einheiten an Aminopeptidase eingesetzt.

Wenn der erwünschte Hydrolysegrade erreicht ist, werden die Peptidasen inaktiviert, zweckmäßig durch kurzzeitiges Erhitzen auf 100°C. Stattdessen kann man auch den pH-Wert auf 3 bis 4 absenken, gegebf. zusätzlich thermisch belasten und gewünschtenfalls nach der Inaktivierung wieder einen höheren pH-Wert einstellen. Das Protein-Hydrolysat kann anschließend in gewünschter Weise zu Nahrungs- oder Futtermitteln verarbeitet werden. Es kann z.B. in flüssiger Form oder nach Sprüh- oder Walzentrocknung in Pulverform weiterverarbeitet werden. Einzelheiten des Hydrolyseverfahrens können dem Teil B der nachfolgenden Beispiele entnommen werden.

### BEISPIELE

### Beispiel 1

### A. Herstellung eines Aminopeptidase-Präparates aus Asp.oryzae-Proteinase

40 g eines Sprühgutes aus A.oryzae wird in 25 g Glycerin und 35 g destilliertem Wasser unter Rühren gelöst. Der pH-Wert wird durch Zusatz von 1 mol. NaOH auf 6,5 gestellt.

Die Lösung wird innerhalb von 45 Minuten auf 70 Grad C erhitzt und dann 30 Minuten bei dieser Temperatur gehalten. Nach Abkühlen werden die Aktivitäten in Anson-Einheiten (AE) pro Gramm bzw. in Pepidase-Einheiten (U) pro Gramm bestimmt und mit dem thermisch nicht belasteten Ausgangsprodukt verglichen:

| | Produkt thermisch | |
|---|---|---|
| | nicht belastet(I) | belastet(II) |
| Proteinaseaktivität pH=7,5 (nach Anson, Substrat = Hemoglobin) | 1,29 AE/g | 0,055 AE/g |
| Pepitdaseaktivität pH=7,0 Substrat = Leucin-p-Nitroanilid) | 384.000 U/g | 373.000U/g |

Die erhaltene Aminopeptidase II wurde in Kombination mit dem thermisch nicht belasteten Ausgangsprodukt I zur Hydrolyse von Gluten eingesetzt.

### B) Hydrolyse von Gluten zur Herstellung eines nichtbitteren Hydrolyseprodukts.

In 2 Vorversuchen wurde der Bitterpunkt bei der Glutenhydrolyse ohne und mit Peptidase bestimmt.

### Versuch im kleinen Maßstab:

Eine 10 %-ige Suspension von Weizengluten wurde auf pH = 8,5 gestellt. Unter Rühren wurden bei 50 Grad C mit je 1 % (bezogen auf Gluten) des Proteinasepräparates I und des Peptidasepräparates II inkubiert, bis das Hydrolysat bitter schmeckt. Dies war nach ca. 2 Std. bei einem (durch Formoltitration ermittelten) Hydrolysegrad von 23,4 % der Fall. Der pH-Wert wurde während der Hydrolyse durch laufende Zugabe von 1 mol Natronlauge konstant gehalten. Der Gluten ging dabei fast vollständig in Lösung.

### Vergleichsversuch ohne Aminopeptidase

Es wurde wie im Vorversuch gearbeitet, nur wurde das Peptidasepräparat weggelassen. Der Bitterpunkt wurde nach ca. 1 Std. bereits bei einem Hydrolysegrad von 15,6 % erreicht. Die Lösung war noch stark trüb. Ohne Exopeptidase liegt also der Bitterpunkt deutlich tiefer.

### Technische Durchführung

Im technischen Maßstab wurde wie im Vorversuch gearbeitet, jedoch wurde kurz vor Erreichen des Bitterpunktes die Hydrolyse durch kurzes Aufkochen abgebrochen. Das Hydrolysat wird sprühgetrocknet und eignet sich als Zusatzstoff für Fleisch und Teigwaren.

### Beispiel 2

### A) Herstellung eines Aminopeptidase-Präparats aus A.sojae-Proteinase.

100 ml eines Ultrafilrats aus einem Kultursaft einer A.sojae-Fermentation werden mit 50 g Sorbit versetzt. Nach dessen Auflösung unter Rühren wird der pH-Wert auf 6,5 gestellt.

Die Lösung wird innerhalb einer Stunde auf 70 Grad C erhitzt und 1 Stunde bei dieser Temperatur gehalten. Nach Abkühlen werden die Aktivitäten bestimmt und mit dem nicht thermisch belasteten Ausgangsprodukt verglichen:

| | Produkt thermisch | |
|---|---|---|
| | nicht belastet (III) | belastet (IV) |
| Proteinaseaktivität, pH=7,5 (nach Anson, Substrat=Hemoglobin) | 0,131 AE/g | 0,0042 AE/g |
| Peptidaseaktiviät, pH=7,0 (Substrat=Leucin-p-Nitroanilid | 52.400 U/mg | 50.100 U/mg |

Die erhaltene Aminopeptidase IV wurde zur Hydrolyse von Molkenprotein in Kombination mit einer Pankreasproteinase (Corolase PP®, Handelsprodukt der Röhm GmbH) eingesetzt. Letztere besitzt bereits einen hohen Anteil an Aminopeptidasen und ergänzt sich vorteilhaft mit dem Produkt IV.

### B) Hydrolyse von Molkenprotein zur Herstellung eines nichtbitteren Hydrolyseprodukts

Es wurde in 2 Vorversuchen der Bitterpunkt bei der Molkenproteinhydrolyse mit und ohne Peptidase bestimmt.

### Vorversuche im kleineren Maßstab

Eine 10 %-ige Molkenproteinlösung wurde auf pH = 7,5 gestellt. Unter Rühren wurden bei 50 Grad C mit 0,9 % (bezogen auf Molkenprotein) der Pankreasproteinase Corolase PP (Röhm GmbH) und 0,4 % des Peptidasepräparats IV inkubiert, bis das Hydrolysat bitter schmeckte. Dies war noch ca. 1 Stunde bei einem (durch Formaltitration bestimmten) Hydrolysegrad von 11,4 % der Fall. Der pH-Wert wurde durch laufende Zugabe von 0,2 mol NaOH konstant gehalten.

### Vergleichsversuch ohne Aminopeptidase

Es wurde wie im Vorversuch gearbeitet, jedoch wurde das Peptidasepräparat weggelassen. Mit Corolase PP allein trat den Bitterpunkt nach ca. 20 Minuten bereits bei einem Hydrolysegrad von 4,2 % auf.

### Hauptansatz im technischen Maßstab

Es wurde wie im Vorversuch gearbeitet, jedoch wurde kurz vor Erreichung des Bitterpunktes die Reaktion durch kurzes Aufkochen abgebrochen. Das Hydrolysat wurde sprühgetrocknet und eignete sich besonders gut als Aufschlagmasse, sowie als Zusatz zu Babynahrung.

### Beispiel 3

### Hydrolyse von Casein zur Herstellung eines nichtbitteren Hydrolyseprodukts

### Vorversuch im kleineren Maßstab

Eine 10%ige Caseinlösung wurde auf pH = 7,5 gestellt. Unter Rühren wurden bei 50 Grad C mit 0,1 % (bezogen auf Casein) des Proteinaseprapärats I und 0,4 % des Peptidasepräparats II (vgl. Beispiel IA) inkubiert, bis das Hydrolysat bitter schmeckte. Dies war nach ca. 1 Stunde bei einem (durch Formoltitration bestimmten) Hydrolysegrad von 4,4 % der Fall. Der pH-Wert wurde durch laufende Zugabe von 0,1 mol NaOH konstant gehalten.

### Vergleichsversuch ohne Aminopeptidase

Es wurde wie im Vorversuch gearbeitet, jedoch wurde das Peptidasepräparat weggelassen. Mit der Proteinase I allein erreicht man den Bitterpunkt nach ca. 20 Minuten bereits bei einem Hydrolysegrad von 1,2 %!

### Hauptansatz im technischen Maßstab

Es wurde wie im Vorversuch gearbeitet, jedoch wurde kurz vor Erreichen des Bitterpunktes die Reaktion durch kurzes Aufkochen abgebrochen. Das Hydrolysat wurde sprühgetrocknet und eignet sich besonders gut als Aufschlagmasse.

## Patentansprüche

1. Verfahren zur Herstellung von Protein-Hydrolysaten mit niedrigem Gehalt an Bitterstoffen durch Einwirkung einer Proteinase und einer mikrobiellen Aminopeptidase auf ein Protein,
dadurch gekennzeichnet,
daß eine Aminopeptidase eingesetzt wird, die aus einem Peptidasen-Gemisch aus einer Aspergillus-Kultur durch mindestens 50 %ige Inaktivierung der begleitenden Peptidasen in einer wäßrigen Lösung mit einem pH-Wert zwischen 5 und 9 bei einer Temperatur von 50 bis 80°C erhalten worden ist.

2. Verfahren nach Anspruch 1, dadurch gekennzeichnet, daß eine Aminopeptidase eingesetzt wird, die durch Inaktivierung in Gegenwart einer wasserlöslichen Polyhydroxyverbindung erhalten worden ist.

3. Verfahren nach Anspruch 2, dadurch gekennzeichnet, daß als wasserlösliche Polyhydroxyverbindung Glycerin, Glucose, Saccharose oder Sorbit eingesetzt worden ist.

4. Verfahren nach Anspruch 1, dadurch gekennzeichnet, daß eine Aminopeptidase aus einer Asp.oryzae-Kultur eingesetzt wird.

5. Verfahren nach Anspruch 1, dadurch gekennzeichnet, daß eine Aminopeptidase eingesetzt wird, die durch Inaktivierung bei pH 6 -7 und einer Temperatur von 60 - 80°C erhalten worden ist.

6. Verfahren nach einem oder mehreren der Ansprüche 1 bis 5, dadurch gekennzeichnet, daß die Protein-Hydrolyse mit einem Gemisch von aus einer Aspergillus-Kultur gewonnenen Proteinasen und Aminopeptidasen durchgeführt wird.

7. Verfahren nach einem oder mehreren der Ansprüche 1 bis 6, dadurch gekennzeichnet, daß die Protein-Hydrolyse über den für die angewandte Proteinase allein geltenden Bitterpunkt hinausgeführt wird.

8. Verfahren nach einem oder mehreren der Ansprüche 1 bis 7, dadurch gekennzeichnet, daß als Protein ein Milchprotein, Sojaprotein oder Gluten eingesetzt wird.

## Claims

1. A process for preparing protein hydrolysates with a low content of bitter substances as a result of a proteinase and a microbial aminopeptidase acting on a protein,
characterized in that,
an aminopeptidase is used which has been obtained from a peptidase mixture from an Aspergillus culture by at least 50% inactivation of the accompanying peptidases in an aqueous solution with a pH value of between 5 and 9 at a temperature of 50 to 80°C.

2. A process according to claim 1, characterized in that an aminopeptidase is used which has been obtained by inactivation in the presence of a water-soluble polyhydroxy compound.

3. A process according to claim 2, characterized in that glycerol, glucose, saccharose or sorbitol are used as a water-soluble polyhydroxy compound.

4. A process according to claim 1, characterized in that an aminopeptidase from an Asp.oryzae culture is used.

5. A process according to claim 1, characterized in that an aminopeptidase is used which has been obtained by inactivation at pH 6 to 7 and at a temperature of 60 to 80°C.

6. A process according to one or more of claims 1 to 5, characterized in that the protein hydrolysis is carried out using a mixture of proteinases and aminopeptidases obtained from an Aspergillus culture.

7. A process according to one or more of claims 1 to 6, characterised in that the protein hydrolysis is carried out beyond the bitter point which is only relevant for the proteinase used.

8. A process according to one or more of claims 1 to 7 characterised in that a milk protein, soya protein, or gluten is used as protein.

## Revendications

1. Procédé de production d'hydrolysats de protéines à faible teneur en principes amers, par action d'une protéinase et d'une aminopeptidase microbienne sur une protéine, caractérisé en ce qu'on utilise une aminopeptidase qui a été obtenue à partir d'une culture d'*Aspergillus* par inactivation à 50% au moins des peptidases accompagnatrices, dans une solution aqueuse ayant un pH compris entre 5 et 9, à une température de 50 à 80°C.

2. Procédé selon la revendication 1, caractérisé en ce qu'on utilise une aminopeptidase qui a été obtenue par inactivation en présence d'un composé polyhydroxylé hydrosoluble.

3. Procédé selon la revendication 2, caractérisé en ce que du glycérol, du glucose, du saccharose ou du sorbitol a été utilisé comme composé polyhydroxylé hydrosoluble.

4. Procédé selon la revendication 1, caractérisé en ce qu'on utilise une aminopeptidase provenant d'une culture d'*Asp*. *oryzae*.

5. Procédé selon la revendication 1, caractérisé en ce qu'on utilise une aminopeptidase qui a été obtenue par inactivation à un pH de 6 à 7 et à une température de 60 à 80°C.

6. Procédé selon l'une quelconque des revendications 1 à 5, caractérisé en ce que l'hydrolyse de la protéine est effectuée avec un mélange de protéinases et d'aminopeptidases obtenues à partir d'une culture d'*Aspergillus*.

7. Procédé selon l'une quelconque des revendications 1 à 6, caractérisé en ce que l'hydrolyse de la protéine est conduite au-delà du point d'amertume observé en cas d'utilisation de la seule protéinase.

8. Procédé selon l'une quelconque des revendications 1 à 7, caractérisé en ce qu'on utilise, comme protéine, une protéine de lait, une protéine de soja ou du gluten.
